(19) Europäisches Patentamt
European Patent Office
Office européen des brevets

(11) Veröffentlichungsnummer: **0 276 466**
**A2**

# (12) EUROPÄISCHE PATENTANMELDUNG

(21) Anmeldenummer: **87119012.0**

(22) Anmeldetag: **22.12.87**

(51) Int. Cl.[4]: **C07D 233/61** , C07D 409/06 , C07D 401/12 , C07D 409/14 , A61K 31/415 , A61K 31/44

Die Bezeichnung der Erfindung wurde geändert (Richtlinien für die Prüfung im EPA, A-III, 7.3).

(30) Priorität: **29.12.86 DE 3644616**

(43) Veröffentlichungstag der Anmeldung: **03.08.88 Patentblatt 88/31**

(84) Benannte Vertragsstaaten: **AT BE CH DE ES FR GB GR IT LI LU NL SE**

(71) Anmelder: **CL PHARMA AKTIENGESELLSCHAFT**
**ST. Peter-Strasse 25**
**A-4021 Linz(AT)**

(72) Erfinder: **Schermanz, Karl, Dr.**
**Gaussgasse 4/10/54**
**A-8010 Graz(AT)**
Erfinder: **Saischek, Gerald, Dipl.-Ing.**
**Roseggerstrasse 4**
**A-4600 Wels(AT)**
Erfinder: **Urmann, Robert**
**Wankmüllerhofstrasse 74**
**A-4020 Linz(AT)**
Erfinder: **Martetschläger, Kurt**
**Himmelbergerstrasse 14**
**A-4020 Linz(AT)**

(74) Vertreter: **Kunz, Ekkehard, Dr.**
**Chemie Holding AG Patentwesen St. Peter-Strasse 25**
**A-4020 Linz(AT)**

(54) **Aminoazolderivate, Verfahren zu deren Herstellung und deren Verwendung als antimykotische Mittel.**

(57) Neue Imidazolderivate der allgemeinen Formel

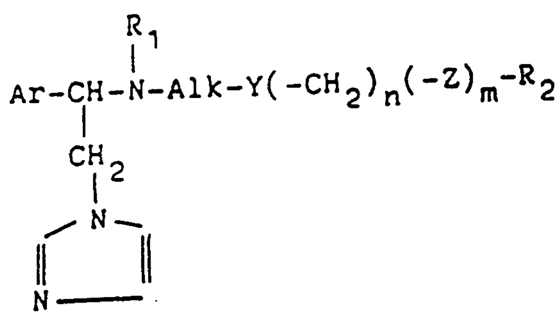

(I)

in welcher Ar gegebenenfalls durch Halogen, Niederalkyl oder Niederalkoxy substituiertes Phenyl, Biphenylyl, Naphthyl oder Thienyl $R_1$ Wasserstoff oder Niederalkyl, Alk geradkettiges oder verzweigtes Alkylen mit

EP 0 276 466 A2

1 bis 10 Kohlenstoffatomen, Y Sauerstoff, Schwefel, Sulfinyl oder Sulfonyl, n eine der Zahlen 0, 1 oder 2, Z Schwefel oder Sulfinyl, m die Zahl 0 oder 1, wobei, wenn Y Schwefel, Sulfinyl oder Sulfonyl bedeutet, m die Zahl 0 und wenn Y Sauerstoff bedeutet, m die Zahl 1 ist, $R_2$ Cyclohexyl, gegebenenfalls durch Hydroxy, Halogen, Trifluormethyl, Niederalkyl oder Niederalkoxy substituiertes Phenyl oder Naphthyl, Biphenylyl oder Pyridyl bedeuten und Verfahren zu ihrer Herstellung. Die Imidazolderivate bei der Anwendung in der Human-und Tiermedizin weisen ausgezeichnete antimykotische Eigenschaften auf.

Die Erfindung betrifft neue Imidazolderivate, Verfahren zu deren Herstellung sowie diese enthaltende Antimykotika und Verfahren zu deren Herstellung.

In der EP-A-183 147 sind $\beta$-substituierte Aminophenethylazolderivate beschrieben, die als Fungizide für Landwirtschaft und Gartenbau eingesetzt werden. Das Bauprinzip der 2-Thioalkylaminoethylimidazole ist jedoch nicht geoffenbart. Aus H. Büchel: Fungicide Chemistry: Advances and Practical Applications, Am.Chem.Soc. Washington 1986, S. 11 - 23 und G. Jäger, Pesticide Chemistry: Human Welfare and the Environment, Vol I, 55 -56, Pergamon Press Oxford 1983 ist bekannt, daß trotz großer struktureller Ähnlichkeit innerhalb der Verbindungsklasse der Azole häufig große Unterschiede in den biologischen Eigenschaften bestehen. Es wurden nun überraschenderweise neue Imidazolderivate gefunden, die bei der Anwendung in der Human-und Tiermedizin ausgezeichnete antimykotische Eigenschaften aufweisen.

Gegenstand der Erfindung sind demnach Imidazolderivate der allgemeinen Formel I des Formelblattes, in welcher Ar gegebenenfalls durch Halogen, Niederalkyl oder Niederalkoxy substituiertes Phenyl, Biphenylyl, Naphthyl oder Thienyl, $R_1$ Wasserstoff oder Niederalkyl, Alk geradkettiges oder verzweigtes Alkylen mit 1 bis 10 Kohlenstoffatomen, Y Sauerstoff, Schwefel, Sulfinyl oder Sulfonyl, n eine der Zahlen 0, 1 oder 2, Z Schwefel oder Sulfinyl, m die Zahl 0 oder 1, wobei, wenn Y Schwefel, Sulfinyl oder Sulfonyl bedeutet, m die Zahl 0 und wenn Y Sauerstoff bedeutet, m die Zahl 1 ist, $R_2$ Cyclohexyl, gegebenenfalls durch Hydroxy, Halogen, Trifluormethyl, Niederalkyl oder Niederalkoxy substituiertes Phenyl oder Naphthyl, Biphenylyl oder Pyridyl bedeuten und deren pharmazeutisch annehmbare Säureadditionssalze.

Weiters wurde gefunden, daß man die erfindungsgemäßen Substanzen erhält, indem man Verbindungen der allgemeinen Formel II des Formelblattes,
worin Ar obige Bedeutung hat, mit Verbindungen der allgemeinen Formel III des Formelblattes,
worin $R_2$, Z, m, n, Y und Alk obige Bedeutung haben, gegebenenfalls in Gegenwart eines inerten Verdünnungsmittels umsetzt, die erhaltenen Iminoverbindungen der allgemeinen Formel IV des Formelblattes,
in welcher Ar, Alk, Y, Z, $R_2$, n und m obige Bedeutung haben, gegebenenfalls in Gegenwart eines inerten Verdünnungsmittels reduziert und gewünschtenfalls die erhaltenen Verbindungen der Formel I, in denen $R_1$ Wasserstoff bedeutet, durch übliche Alkylierungsmethoden in Verbindungen der Formel I, in denen $R_1$ Niederalkyl bedeutet, überführt.

Die Umsetzung der Verbindungen II und III erfolgt z. B. durch Erhitzen des Reaktionsgemisches in einem organischen Verdünnungsmittel. Werden Verbindungen III in Form ihrer Salze eingesetzt, so ist die Zugabe eines Äquivalentes einer Base, wie Trialkylamin, Natriumalkoholat oder Alkalihydroxyd erforderlich.

Als Verdünnungsmittel dienen aliphatische oder aromatische Kohlenwasserstoffe, die chloriert sein können, wie Benzinfraktionen, Perchloräthylen, Benzol, Toluol, Chlorbenzol, Xylol, Ether wie Dibutylether, Dioxan, Alkohole wie Butanol, Pentanol, Ethylenglykol, Säureamide wie Dimethylformamid und Mischungen derselben mit den oben genannten Verdünnungsmitteln. Die Komponenten werden am Wasserabscheider unter Rückfluß erhitzt bis sich kein Reaktionswasser mehr abscheidet. Die nach Entfernen des Verdünnungsmittels erhaltene Iminoverbindung IV wird in einem organischen Verdünnungsmittel gelöst bzw. suspendiert und gekühlt. Als Verdünnungsmittel dienen insbesondere Alkohole, vorzugsweise Methanol, Ether wie Diethylether oder Tetrahydrofuran. Die Reduktion erfolgt dann durch Zugabe eines Reduktionsmittels, insbesondere eines komplexen Metallhydrides wie z. B. Alkaliborhydrid, Alkalicyanoborhydrid, Aluminiumborhydrid, Lithiumaluminiumhydrid, vorzugsweise Natriumborhydrid bei etwa einer Temperatur zwischen -20 °C und der Rückflußtemperatur des verwendeten Verdünnungsmittels, vorzugsweise bei einer Temperatur von -5 °C bis +20 °C.

Für die Einführung des Alkylrestes $R_1$ kommen alle gebräuchlichen Alkylierungsmethoden in Frage. Zur Einführung des Methylrestes kann beispielsweise eine Verbindung der Formel I, in der $R_1$ Wasserstoff bedeutet, in alkoholischer, z. B. methanolischer, Lösung mit wässriger Formaldehydlösung versetzt, das Gemisch zum Sieden erhitzt und auf die Reaktionslösung nach ihrem Erkalten ein Reduktionsmittel, vorzugsweise Natriumborhydrid einwirken gelassen werden.

Nach einem weiteren Verfahren werden die erfindungsgemäßen Substanzen erhalten, indem man eine Verbindung der allgemeinen Formel II mit einem Aminoalkanol der allgemeinen Formel V des Formelblattes,
umsetzt, die als Umsetzungsprodukt erhaltene Iminoverbindung der allgemeinen Formel VI des Formelblattes,
reduziert, die erhaltene Hydroxylalkylamino-Verbindung der allgemeinen Formel Formel VII des Formelblattes,
in die entsprechende Halogenalkylamino-Verbindung der allgemeinen Formel VIII des Formelblattes,
überführt, diese mit einer Verbindung der allgemeinen Formel IX des Formelblattes,
umsetzt und gewünschtenfalls die erhaltenen Verbindungen der Formel I, in denen $R_1$ Wasserstoff bedeutet,

durch übliche Alkylierungsmethoden in Verbindung der Formel I, in denen $R_1$ Niederalkyl bedeutet, überführt, wobei in obigen Formeln V bis IX Ar, Alk, Y, Z, $R_2$, n und m die für Formel I angegebene Bedeutung haben und Hal für Halogen steht.

Die Umsetzung einer Verbindung der allgemeinen Formel II mit einem Aminoalkohol der allgemeinen Formel V wird in einem organischen Verdünnungsmittel bei einer Temperatur zwischen 0 °C und 180 °C, vorzugsweise bei Rückflußtemperatur des verwendeten Verdünnungsmittels durchgeführt. Als Verdünnungsmittel dienen aliphatische oder aromatische Kohlenwasserstoffe die chloriert sein können, wie Benzinfraktionen, Perchloräthylen, Benzol, Toluol, Xylol, Chlorbenzol, Ether wie Dibutylether, Dioxan, Alkohole wie Butanol, Pentanol, Ethylenglykol, Säureamide wie Dimethylformamid und Mischungen desselben mit den oben angeführten Verdünnungsmitteln. Die nach Entfernen des Verdünnungsmittels erhaltene Iminoverbindung VI wird in einem organischen Verdünnungsmittel gelöst bzw. suspendiert und die Lösung bzw. Suspension gekühlt. Als Verdün nungsmittel dienen insbesondere Alkohole, vorzugsweise Methanol, Ether wie Diethylether oder Tetrahydrofuran. Die Reduktion erfolgt durch Zugabe eines Reduktionsmittels, vorzugsweise eines komplexen Metallhydrides, insbesondere Natriumborhydrid bei einer Temperatur von etwa -20 °C bis Rückflußtemperatur des verwendeten Verdünnungsmittels, vorzugsweise bei einer Temperatur von etwa -5 °C bis +20 °C. Die nach üblicher Aufarbeitung erhaltene Hydroxyalkylamino-Verbindung VII wird in einem organischen Verdünnungsmittel, vorzugsweise in einem chlorierten aliphatischen Kohlenwasserstoff, z. B. Chloroform, gelöst und die Lösung gekühlt. Durch Zugabe eines Halogenierungsmittels, z. B. Phosphortribromid, erfolgt die Überführung der Hydroxyverbindung VII in die entsprechende Halogenverbindung VIII, wobei die Reaktion bei einer Temperatur von etwa -50 °C bis Raumtemperatur, vorzugsweise von -20 °C bis 0 °C durchgeführt wird. Die Umsetzung der Halogenalkylamino-Verbindung VIII mit der Verbindung der allgemeinen Formel IX erfolgt vorzugsweise in alkoholischer, z. B. methanolischer, Lösung in Gegenwart einer Base, z. B. Natriummethylat oder Alkalihydroxyd bei einer Temperatur von etwa -20 °C bis 120 °C, vorzugsweise bei einer Temperatur von 20 °C bis 80 °C.

In den Formeln I bis IX bedeuten $R_1$ ein Wasserstoffatom oder einen Alkylrest mit 1 bis 4 C-Atomen, vorzugsweise ein Wasserstoffatom oder den Methylrest. Ar bedeutet durch Halogen, Niederalkyl oder Niederalkoxy substituiertes Phenyl, Biphenylyl, Naphthyl oder Thienyl, vorzugsweise 2,4-Dichlorphenyl.

Alk bedeutet einen geradkettigen oder verzweigten gesättigten Kohlenwasserstoffrest mit 1 bis 10 C-Atomen. Beispiele für solche Reste sind Methyl-, Ethyl-, n-Propyl-, i-Propyl-, Butyl-, s-Butyl-, t-Butylreste, geradkettige oder verzweigte Pentyl-, Hexyl-, Heptyl-und Octylreste.

Y bedeutet Schwefel, Sulfinyl oder Sulfonyl, besonders bevorzugt Schwefel, sowie Sauerstoff wenn m gleich 1 ist.

Z kann Schwefel oder Sulfinyl bedeuten.

$R_2$ bedeutet einen Cyclohexylrest, Phenyl oder Naphthylreste, die ein-oder mehrfach durch Halogenatome, Hydroxylgruppen, Alkyl-oder Alkoxyreste mit 1-4 C-Atomen oder Trifluormethyl substituiert sein können, Biphenylyl oder Pyridyl, vorzugsweise 4-Chlorphenyl, 4-Bromphenyl, Cyclohexyl oder Naphthyl.

Die erfindungsgemäßen Verbindungen und ihre pharmakologisch verträglichen Salze besitzen interessante antimykotische Eigenschaften und können als Heilmittel in der Human-und Tiermedizin verwendet werden. Diese Wirkung konnte durch Bestimmung der minimalen Hemmstoffkonzentration (MHK) bei Hefen, Schimmelpilzen und Dermatophyten nachgewiesen werden.

Die erfindungsgemäßen Wirkstoffe können in üblicher Weise als feste, halbfeste oder flüssige Formulierungen in Form von Tabletten, Kapseln, Pulvern, Suppositorien, Lösungen, Cremes, Lotionen, Gels, Salben oder dgl. angewendet werden. Pharmazeutisch verträgliche nicht toxische Träger oder Exzipienten, die normalerweise für feste Formulierungen verwendet werden, sind beispielsweise Tricalcium-phosphat, Calciumcarbonat, Kaolin, Bentonit, Talkum, Gelatine, Lactose, Stärke Für halbfeste Formulierungen eignen sich beispielsweise Wasser, pflanzliche Öle und niedrigsiedende Lösungsmittel, wie i-Propanol, hydrierte Naphthaline u. dgl.

Die die erfindungsgemäßen Wirkstoffe enthaltenden pharmazeutischen Mittel können herkömmlichen pharmazeutischen Maßnahmen, wie der Sterilisierung unterzogen werden und können herkömmliche pharmazeutische Exzipienten wie Konservierungsmittel, Stabilisierungsmittel, Emulgatoren, Salze zur Einstellung des osmotischen Druckes und Puffer enthalten. Die Mittel können neben den erfindungsgemäßen Verbindungen auch andere therapeutische wirksame Materialien enthalten.

Die die erfindungsgemäßen Verbindungen enthaltenden Mittel bestehen normalerweise aus einem pharmazeutisch verträglichen nicht toxischen Träger in Verbindung mit einer oder mehreren erfindungsgemäßen Verbindungen in einer wirksamen Menge, die zur Erleichterung oder Verhütung der spezifischen zu behandelnden Zustände führt. Da die erfindungsgemäßen Wirkstoffe über einen weiten Konzentrationsbereich antimykotische Wirkung zeigen, kann die wirksame Menge variieren. Bei topischen Formulierungen kann die Menge beispielsweise ungefähr 0,1 bis 10 % der gesamten pharmazeutischen Formulierung

betragen, wohingegen bei anderen Formulierungen die Menge ungefähr 5 bis etwa 95 % oder mehr ausmachen kann. Vorzugsweise werden die erfindungsgemäßen pharmazeutischen Mittel als Dosiseinheit formuliert um die Applikation zu erleichtern.

Die erfindungsgemäßen Verbindungen und Mittel können zur pharmazeutischen Anwendung bei Menschen und Tieren auf herkömmliche Weise z. B.: topisch, oral, parenteral oder in ähnlicher Weise verabreicht weerden. Die genaue Vorschrift zur pharmazeutischen Applikation der erfindungsgemäßen Verbindungen und Mittel hängt notwendigerweise von den Anforderungen des Einzelfalls, der Art der Behandlung, die beispielsweise vorbeugend oder heilend sein kann, der Art der beteiligten Organismen ab.

Zur systemischen, beispielsweise oralen oder parenteralen Applikation ist es im allgemeinen angebracht, den Wirkstoff in Mengen von etwa 1 - 120 mg/kg Körpergewicht pro Tag, vorzugsweise 5 - 100 mg/kg Körpergewicht pro Tag zu verabreichen, wobei diese Mengen auch auf mehrere Gaben (beispielsweise 3 pro Tag) verteilt werden können um gute Resultate zu erzielen. Zur lokalisierten Applikation ist jedoch entsprechend weniger Wirkstoff erforderlich.

Beispiel 1 (Verbindung Nr. 23):

a) Herstellung des Zwischenproduktes

1-(2-(2,4-Dichlorphenyl)-2-(3-(4-bromphenylthio)propylimino))ethyl-1H-imidazol

14.8 g (0.058 Mol) 2,4-Dichlorphenacylimidazol, 16.8 g (0.059 Mol) 4-Bromphenylthiopropylaminhydrochlorid und 6.0 g (0.059 Mol) Triethylamin werden in 100 ml Toluol suspendiert bzw. gelöst und das Gemisch am Wasserabscheider unter Rückfluß erhitzt, bis sich kein Reaktionswasser mehr abscheidet. Danach wäscht man die Reaktionslösung mit Wasser, trocknet die org. Phase mit Natriumsulfat und erhält nach dem Abdampfen des Lösungsmittels 27.9 g 1-(2-(2,4-Dichlorphenyl)-2-(3-(4-bromphenylthio)-propylimino))ethyl-1H-imidazol als viskoses Öl. (Ausbeute: 98 %)

b) Herstellung des Endproduktes

1-(2-(2,4-Dichlorphenyl)-2-(3-(4-bromphenylthio)propylamino))ethyl-1H-imidazol

27.9 g (0.057 Mol) 1-(2-(2,4-Dichlorphenyl)-2-(3-(4-bromphenylthio)propylimino))ethyl-1H-imidazol werden in 150 ml Methanol gelöst, die Lösung auf -5 °C gekühlt und portionsweise 6.4 g (0.169 Mol) Natriumborhydrid so zugefügt, daß die Temperatur nicht über 5 °C steigt. Anschließend rührt man noch 1 Stunde bei 30 °C, dampft hierauf zur Trockene ein und bringt das Reaktionsgemisch mit halbkonzentrierter Salzsäure auf pH = 1. In weiterer Folge wird die Reaktionslösung mit 40%iger Natronlauge auf pH = ca. 12 gebracht und mehrmals mit Dichlormethan extrahiert. Nach Waschen der vereinigten Extrakte mit Wasser, Trocknen und Entfernen des Lösungsmittels im Vakuum erhält man ein Öl, aus dem durch Behandlung mit Aceton und Salpetersäure 10.6 g reines Dinitrat des 1-(2-(2,4-Dichlorphenyl)-2-(3-(4-bromphenylthio)propylamino)ethyl-1H-imidazols mit dem Schmp. 162 -179 °C erhalten werden. (Ausbeute: 32 %)

Beispiel 2 (Verbindung Nr. 20):

a) Herstellung des Zwischenproduktes

1-(2-(2,4-Dichlorphenyl)-2-(3-hydroxypropylimino))ethyl-1H-imidazol

153.2 g (0.60 Mol) N-(2,4-Dichlorphenacyl)-imidazol und 53.0 g (0.705 Mol) 3-Amino-1-propanol werden in 400 ml Toluol suspendiert bzw. gelöst und das Gemisch am Wasserabscheider unter Rückfluß erhitzt, bis sich kein Reaktionswasser mehr abscheidet. Danach wäscht man die Reaktionslösung 3 Mal mit Wasser, trocknet die organische Phase mit Natriumsulfat und erhält nach dem Abdampfen des Lösungsmittels 179 g 1-(2-(2,4-Dichlorphenyl)-2-(3-hydroxypropylimino))ethyl-1H-imidazol als hochviskoses Öl.
(Ausbeute: 95.6 %)

1-(2-(2,4-Dichlorphenyl)-2-(3-hydroxypropylamino))ethyl-1H-imidazol

179.0 g (0.5737 Mol) 1-(2-(2,4-Dichlorphenyl)-2-(3-hydroxypropylimino))ethyl-1H-imidazol werden in 300 ml Methanol gelöst, die Lösung auf 0 °C gekühlt und portionsweise 50.0 g (1.322 Mol) Natriumborhydrid so zugefügt, daß die Temperatur nicht über 5 °C steigt. Nach Zugabe des Borhydrids rührt man noch 2 Stunden bei Raumtemperatur, dampft anschließend zur Trockene ein und bringt das Reaktionsgemisch mit halbkonzentrierter Salzsäure auf pH = 1. In weiterer Folge wird die Reaktionslösung mit 40%iger Natronlauge auf pH = ca. 12 gebracht und mehrmals mit Dichlormethan extrahiert. Nach Waschen der vereinigten organischen Extrakte mit Wasser, Trocknen und Entfernen des Lösungsmittels im Vakuum, erhält man 169 g Rohprodukt als Öl. Nach Umkristallisation des Öles aus Aceton werden 107 g reines 1-(2-(2,4-Dichlorphenyl)-2-(3-hydroxypropylamino))ethyl-1H-imidazol vom Schmp. 77 -79 °C erhalten. (Ausbeute: 51 %)

1-(2-(2,4-Dichlorphenyl)-2-(3-brompropylamino)) ethyl-1H-imidazol

12.6 g (0.04 Mol) 1-(2-(2,4-Dichlorphenyl)-2-(3-hydroxypropylamino))ethyl-1H-imidazol werden in 30 ml Chloroform gelöst und die Lösung auf -5 °C gekühlt. Unter Rühren werden langsam 10.83 g Phosphortribromid, gelöst in 20 ml $CHCl_3$, so zugetropft, daß die Temperatur nicht über 0 °C steigt. Nach dem Zutropfen versetzt man das Reaktionsgemisch mit 100 ml Petrolether, wobei 20.5 g kristallines 1-(2-(2,4-Dichlorphenyl)-2-(3-brompropylamino))ethyl-1H-imidazol als Dihydrobromid vom Schmp. 140 - 150 °C erhalten werden, welches aus Stabilitätsgründen sofort weiter umgesetzt wird. (Ausbeute: 95 %)

b) Herstellung des Endproduktes

1-(2-(2,4-Dichlorphenyl)-2-(3-(4-chlorphenylthio)propylamino))ethyl-1H-imidazol

5.4 g (0.01 Mol) frisch bereitetes 1-(2-(2,4-Dichlorphenyl)-2-(3-brompropylamino))ethyl-1H-imidazoldihydrobromid und 1.45 g (0.01 Mol) 4-Chlorthiophenol werden in 50 ml Methanol gelöst und mit 6 ml einer 30%igen Natriummethylatlösung versetzt. Man erhitzt das Reaktionsgemisch 2 Stunden auf Rückfluß und rührt anschließend noch 14 Stunden bei Raumtemperatur. In der Folge verdampft man das Methanol im Vakuum, nimmt den Rückstand in Dichlormethan auf, schüttelt mit 5%iger Natronlauge und wäscht die organische Phase mit Wasser. Nach Trocknen und Entfernen des Lösungsmittels im Vakuum löst man den Rückstand in Aceton und versetzt tropfenweise mit konzentrierter Salpetersäure, wobei 3.0 g 1-(2-(2,4-Dichlorphenyl)-2-(3-(4-chlorphenylthio)propylamino))ethyl-1H-imidazol als Dinitrat anfallen. Aus Alkohol umkristallisiert erhält man 2.2 g farblose Kristalle vom Schmp. 168 - 177 °C.
(Ausbeute: 41 %)

Beispiel 3 (Verbindung Nr. 36):

Herstellung der N-Alkylverbindungen

1-(2-(2,4-Dichlorphenyl)-2-(N-methyl-N-(3-(4-chlorbenzylthio)propylamino)))ethyl-1H-imidazol

8.18 g (0.018 Mol) 1-(2-(2,4-Dichlorphenyl)-2-(3-(4-chlorbenzylthio)propylamino))ethyl-1H-imidazol werden in 100 ml Methanol gelöst und mit 34.3 g 35%iger wässriger Formaldehydlösung versetzt und das Gemisch 2 Stunden zum Sieden erhitzt. Nach dem Erkalten der Reaktionslösung werden 14.6 g Natriumborhydrid zugesetzt und bei Raumtemperatur 14 Stunden gerührt. In der Folge verdampft man das Methanol im Vakuum, versetzt den Rückstand mit halbkonzentrierter Salzsäure, fügt anschließend bis pH = 12 40%ige Natronlauge zu und extrahiert 3 Mal mit Dichlormethan. Die vereinigten Extrakte werden mit Wasser gewaschen und anschließend das Lösungsmittel im Vakuum verdampft, wobei ein Öl anfällt. Das Rohprodukt wird über Kieselgel chromatographiert (Laufmittel: Essigester/Methanol = 10 : 1). Man erhält ein Öl, aus welchem durch Behandlung mit ethanolischer Salzsäure 2.0 g 1-(2-(2,4-Dichlorphenyl)-2-(N-methyl-N-(3-(4-chlorbenzylthio)propylamino)))ethyl-1H-imidazol als Dihydrochlorid vom Schmp. 170 - 180 °C erhalten werden. (Ausbeute: 21 %)

Nach einem der angegebenen Verfahren wurden folgende Verbindungen erhalten:

Tabelle 1

| Nr | Ar | $R_1$ | Alk | Y | n | Z | $R_2$ | Salz | Fp°C |
|---|---|---|---|---|---|---|---|---|---|
| 1 | 4-Chlor-phenyl | H | $-(CH_2)_2-$ | S | - | - | 4-Chlor-phenyl | 2HCl. $H_2O$ | 124-127 |
| 2 | 2,4-Dichlor-phenyl | H | $-(CH_2)_2-$ | S | - | - | Cyclo-hexyl | $2HNO_3$ | 149-160 |
| 3 | 2,4-Dichlor-phenyl | H | $-(CH_2)_2-$ | S | - | - | 4-Chlor-phenyl | 2HCl | 206-212 |
| 4 | 2,4-Dichlor-phenyl | H | $-(CH_2)_2-$ | S | - | - | 4-Brom-phenyl | $2HNO_3$ | 183-186 |
| 5 | 2,4-Dichlor-phenyl | H | $-(CH_2)_2-$ | S | - | - | 4-Metho-xyphenyl | $2HNO_3$ | 163-174 |
| 6 | 2,4-Dichlor-phenyl | H | $-(CH_2)_2-$ | S | - | - | 2-Naphthyl | $2HNO_3$ | 188-195 |
| 7 | 2,4-Dichlor-phenyl | H | $-(CH_2)_2-$ | S | 1 | - | Phenyl | $2H_2C_2O_4$. $H_2O$ | 156 |
| 8 | 2,4-Dichlor-phenyl | H | $-(CH_2)_2-$ | S | 1 | - | Phenyl | - | zähes Öl |
| 9 | 2,4-Dichlor-phenyl | H | $-(CH_2)_2-$ | S | 1 | - | 4 Chlor-phenyl | $2HNO_3$ | 157-161 |
| 10 | 2,4-Dichlor-phenyl | H | $-(CH_2)_2-$ | S | 1 | - | 3-Trifluor-methyl-phenyl | - | zähes Öl |
| 11 | 4-Brom-phenyl | H | $-(CH_2)_3-$ | S | - | - | 4-Chlor-phenyl | - | zähes Öl |
| 12 | 4-Methyl-phenyl | H | $-(CH_2)_3-$ | S | - | - | 4-Chlor-phenyl | - | zähes Öl |
| 13 | 4-Methoxy-phenyl | H | $-(CH_2)_3-$ | S | - | - | 4-Chlor-phenyl | - | zähes Öl |
| 14 | 4-Biphenylyl | H | $-(CH_2)_2-$ | S | - | - | 4-Chlor-phenyl | 2HCl | 190-197 |
| 15 | 2,4-Dichlor-phenyl | H | $-(CH_2)_3-$ | S | - | - | Cyclo-hexyl | $2HNO_3$ | 170-174 |

| Nr | Ar | $R_1$ | Alk | Y | n | Z | $R_2$ | Salz | Fp. |
|---|---|---|---|---|---|---|---|---|---|
| 16 | 2,4-Dichlor-phenyl | H | $-(CH_2)_3-$ | S | - | - | Phenyl | 2HCl. $H_2O$ | Harz |
| 17 | 2,4-Dichlor-phenyl | H | $-(CH_2)_3-$ | S- | - | - | 4-Methyl-phenyl | $2HNO_3$ | 196-200 |
| 18 | 2,4-Dichlor-phenyl | H | $-(CH_2)_3-$ | S | - | - | 4-Fluor-phenyl | $2HNO_3$ | 160-165 |
| 19 | 2,4-Dichlor-phenyl | H | $-(CH_2)_3-$ | S | - | - | 4-Chlor-phenyl | 2HCl | 193-200 |
| 20 | 2,4-Dichlor-phenyl | H | $-(CH_2)_3-$ | S | - | - | 4-Chlor-phenyl | $2HNO_3$ | 168-177 |
| 21 | 2,4-Dichlor-phenyl | H | $-(CH_2)_3-$ | SO | - | - | 4-Chlor-phenyl | - | zähes Öl |
| 22 | 2,4-Dichlor-phenyl | H | $-(CH_2)_3-$ | $SO_2$ | - | - | 4-Chlor-phenyl | - | zähes Öl |
| 23 | 2,4-Dichlor-phenyl | H | $-(CH_2)_3-$ | S | - | - | 4-Brom-phenyl | $2HNO_3$ | 162-179 |
| 24 | 2,4-Dichlor-phenyl | H | $-(CH_2)_3-$ | S | - | - | 4-Brom-phenyl | 2HCl | 178-187 |
| 25 | 2,4-Dichlor-phenyl | H | $-(CH_2)_3-$ | S | | | 4-Phenolyl | 2HCl | 202-216 |
| 26 | 2,4-Dichlor-phenyl | H | $-(CH_2)_3-$ | S | - | - | 2,6-Dichlor phenyl | $2HNO_3$ | 180-190 |
| 27 | 2,4-Dichlor-phenyl | H | $-(CH_2)_3-$ | S | - | - | 2-Naphthyl | $2HNO_3$ | 196 |
| 28 | 2,4-Dichlor-phenyl | H | $-(CH_2)_3-$ | S | - | - | 2-Pyridyl | - | zähes Öl |
| 29 | 2,4-Dichlor-phenyl | H- | $-(CH_2)_3-$ | S- | - | - | 2-Pyridyl | $2HNO_3$ | 178-179 |
| 30 | 2,4-Dichlor-phenyl | H- | $-(CH_2)_3-$ | S | - | - | 4-Pyridyl | 3HCl. 2,5$H_2O$ | zerfl. Harz |
| 31 | 2-Thienyl | H | $-(CH_2)_3-$ | S | - | - | 4-Chlor-phenyl | - | zähes Öl |
| 32 | 1-Naphthyl | H | $-(CH_2)_3-$ | S | - | - | 4-Fluor-phenyl | HCl | 153-156 |

| Nr | Ar | $R_1$ | Alk | Y | n | Z | $R_2$ | Salz | Fp. |
|---|---|---|---|---|---|---|---|---|---|
| 33 | 1-Naphthyl | $CH_3$ | $-(CH_2)_3-$ | S | - | - | 4-Fluor-phenyl | 2HCl | 175-190 |
| 34 | 2,4-Dichlor-phenyl | H | $-(CH_2)_3-$ | S | 1 | - | Phenyl | $2HNO_3$ | 128-137 |
| 35 | 2,4-Dichlor-phenyl | H | $-(CH_2)_3-$ | S | 1 | - | 4-Chlor-phenyl | $2HNO_3$ | 108-133 |
| 36 | 2,4-Dichlor-phenyl | $CH_3$ | $-(CH_2)_3-$ | S | 1 | - | 4-Chlor-phenyl | 2HCl | 170-180 |
| 37 | 2,4-Dichlor-phenyl | H | Butylen(2) | S | - | - | 4-Chlor-phenyl | $2HNO_3$ | 143-147 |
| 38 | 2,4-Dichlor-phenyl | H | $-(CH_2)_5-$ | S | - | - | 4-Chlor-phenyl | $2HNO_3$ | 156-161 |
| 39 | 2,4-Dichlor-phenyl | H | $-(CH_2)_2-$ | O | 2 | S | 4-Chlor-phenyl | $2HNO_3$ | 81-85 |
| 40 | 2,4-Dichlor-phenyl | H | $-(CH_2)_2-$ | O | 2 | SO | 4-Chlor-phenyl | - | Harz |
| 41 | 1-Naphthyl | H | $-(CH_2)_3$ | S | - | - | 4-Chlor-phenyl | HCl | 172-176 |

Beispiel A

200 mg Wirkstoff enthaltende Tablette zur peroralen Applikation

2 g der Verbindung Nr. 19 und 1 g Milchzucker wurde mit 1 ml 10%iger wässriger Polyvinylpyrrolidon K25-Lösung granuliert. Das Gemisch wurde durch ein Sieb der Maschenweite 3 - 5 mm geschlagen und getrocknet. Dieses getrocknete Gemisch durch ein Sieb der Maschenweite 0,8 - 1,25 mm homogenisiert und dann mit 0,58 g mikrokristalliner Cellulose (Avicel PH102), 30 mg Na-Carboxymethylstärke und 2 mg Magnesiumstearat vermischt. Die so erhaltene Mischung wurde zu 10 Tabletten verpreßt.

Beispiel B

1%ige Lösung zur lokalen Behandlung

Eine Lösung von 1 g der Verbindung Nr. 19 in 50 ml aquapurificata wurde mit soviel Polyethylenglycol 400 versetzt, daß insgesamt 100 ml Lösung entstanden.

Beispiel C

1% Salbe zur lokalen Behandlung

66 g Vaseline liquid. wurden am Wasserbad mit 3,5 g Alfol 16 (Cetylalkohol) und 0,1 g Cholesterin aufgeschmolzen und mit einer Lösung von 1 g der Verbindung Nr. 19 in 29,4 g aqua purificata versetzt. Unter langsamen Abkühlen wurde diese Mischung zu 100 g Salbe homogenisiert.

Beispiel D

1% Injektionslösung (Ampullen zu 100 mg Wirkstoff)

3 g der Verbindung Nr. 19 und 0,3 g eines Gemisches aus 2 Teilen p-Hydroxybenzoesäuremethylester und einem Teil p-Hydroxybenzoesäurepropylester wurden mit aqua ad injectionen ad 300 ml gelöst und durch ein Membranfilter der Porenweite 0,2 µm steril und partikelfrei filtriert, dann unter aseptischen Bedingungen in Ampullen zu 10 ml abgefüllt.

Beispiel E:

Die Ermittlung der antimykotischen Aktivitäten der Verbindungen erfolgte in vitro durch Bestimmung der minimalen Hemmstoffkonzentration (MHK) bei Hefen, Schimmelpilzen und Dermatophyten.

Für die Testung mit Pilzen wurden 6 Dermatophyten, 2 Hefen und 4 Schimmelpilze verwendet:

Trichophyton mentagrophytes (Tri.me.)
Trichophyton rubrum (Tri.ru.)
Trichophyton verrucosum (Tri.ve.)
Microsporum canis (Mi.can.)
Epidermophyton floccosum (Ep.flo.)
Microsporum gypseum (Mi.gyp.)
Candida albicans (C.alb.)
Candida tropicalis (C.trop.)
Aspergillus fumigatus (Asp.fu.)
Mucor mucedo plus (Mu.mu+)
Mucor mucedo minus (Mu.mu-)
Absidia ramosa (Abs.ra.)

Die Bestimmung der minimalen Hemmstoffkonzentration (MHK) erfolgte in Reihenverdünnungstests in Reagenzgläsern. Das Volumen des flüssigen Nährmediums betrug 4,5 ml pro Reagenzglas.

Die Substanzen wurden in DMSO gelöst und mit sterilem destillierten Wasser auf 10 Konzentrationen (100, 50, 25, 12.5, 6.25, 3.12, 1.56, 0.78, 0.39 und 0.19 µg/ml) verdünnt. Aus diesen Verdünnungsstufen wurden jeweils 0.5 ml dem flüssigen Nährmedium zugesetzt. Somit war auch eine einheitliche Konzentration von Lösungsmittel in allen Nährmedien unabhängig von der Wirkstoffkonzentration gewährleistet.

Eine Vergleichslösung, die nur das Lösungsmittel in entsprechender Konzentration enthielt, wurde bei der Durchführung des Tests jeweils mitberücksichtigt.

Die einzelnen Stämme wurden auf Sabouraud-Bierwürze-Schrägager gewartet, und machten vor ihrem Testeinsatz eine Passage auf einem modifizierten Sabouraud-Flüssig-Nährboden durch. Anschließend wurden die Stämme geerntet, gewaschen und zu einer Suspension von McFaerland 3 bei den Hefen und Schimmelpilzen und McFaerland 4 - 5 bei den Dermatophyten aufbereitet.

Die Menge des Impfmaterials (Inoculum) betrug 100 µl/Reagenzglas (Einsaatdichten: Hefen ca. $10^3$/ml, Schimmelpilze ca. $10^4$/ml, Dermatophyten ca. $10^4$/ml). Der pH-Wert des Flüssignährbodens betrug 6.0. Nach erfolgter Inoculation wurden die Pilze bei 22 °C 14 Tage bebrütet.

Anschließend wurde der MHK-Wert bestimmt. Für die Bestimmung des MHK-Wertes wurde jene Konzentrationsstufe herangezogen bei welcher makroskopisch kein sichtbares Wachstum mehr festgestellt werden konnte.

Als Vergleichssubstanz diente 1-(2-(2,4-Dichlorphenyl)-2-((2,4-dichlorphenyl)methoxy-ethyl)-1-H-imidazol als Nitrat (Verbindung A)

TABELLE II MHK-Werte (µg/ml)

| Verbindung Nr. | Tri. me. | Tri. ru. | Tri. ve. | Mi. can | Ep. flo | Mi. gyp | C. alb | C. trop | Asp. fu. | Mu. mu+ | Mu. mu- | Abs. ra. |
|---|---|---|---|---|---|---|---|---|---|---|---|---|
| 3 | 0,78 | 0,78 | 0,78 | 0,78 | 0,78 | 0,78 | 3,12 | 3,12 | 6,25 | 3,12 | 3,12 | 6,25 |
| 4 | 1,56 | 1,56 | 1,56 | 6,25 | 1,56 | 0,78 | 6,25 | 6,25 | 6,25 | 6,25 | 6,25 | 12,5 |
| 5 | 6,25 | 6,25 | 6,25 | 12,5 | 12,5 | 6,25 | 25,0 | 12,5 | 12,5 | 12,5 | 12,5 | 25,0 |
| 6 | 6,25 | 3,12 | 3,12 | 3,12 | 6,25 | 3,12 | 6,25 | 6,25 | 12,5 | 12,5 | 12,5 | 12,5 |
| 16 | 25,0 | 1,56 | 1,56 | 3,12 | 3,12 | 3,12 | 6,25 | 3,12 | 3,12 | 6,25 | 3,12 | 12,5 |
| 17 | 0,78 | 0,78 | 0,78 | 3,12 | 0,78 | 0,78 | 12,5 | 12,5 | 0,78 | 0,78 | 3,12 | 6,25 |
| 18 | 6,25 | 0,78 | 0,39 | 6,25 | 3,12 | 3,12 | 12,5 | 6,25 | 12,5 | 12,5 | 12,5 | 6,25 |
| 19 | 0,39 | 0,19 | 0,39 | 0,39 | 0,19 | 0,39 | 0,39 | 0,78 | 0,78 | 1,56 | 1,56 | 1,56 |
| 20 | 12,5 | 0,19 | 0,39 | 1,56 | 6,25 | 3,12 | 3,12 | 1,56 | 3,12 | 12,5 | 25,0 | 3,12 |
| 23 | 0,39 | 0,39 | 0,30 | 1,56 | 0,78 | 0,39 | 3,12 | 1,56 | 1,56 | 6,25 | 6,25 | 3,12 |
| 26 | 3,12 | 3,12 | 3,12 | 12,5 | 3,12 | 3,12 | 6,25 | 6,25 | 12,5 | 6,25 | 12,5 | 12,5 |
| 27 | 3,12 | 1,56 | 1,56 | 0,78 | 3,12 | 3,12 | 3,12 | 6,25 | 0,78 | 6,25 | 3,12 | 6,25 |
| 36 | 6,25 | 6,25 | 6,25 | 1,56 | 1,56 | 6,25 | 3,12 | 0,78 | 25,0 | 6,25 | 6,25 | 50,0 |
| 38 | 0,78 | 3,12 | 6,25 | 12,5 | 0,78 | 3,12 | 6,25 | 3,12 | 6,25 | 0,78 | 3,12 | 6,25 |
| 6 | 6,25 | 3,12 | 3,12 | 12,5 | 3,12 | 3,12 | 6,25 | 25 | 6,25 | 12,5 | 25 | 12,5 |
| 11 | 6,25 | 3,12 | 6,25 | 6,25 | 6,25 | 6,25 | 3,12 | 12,5 | 6,25 | 12,5 | 25 | 6,25 |
| 24 | 0,10 | 0,10 | 0,10 | 0,78 | 0,10 | 0,39 | 3,12 | 3,12 | 0,39 | 3,12 | 3,12 | 12,5 |
| 14 | 0,78 | 1,56 | 0,78 | 6,25 | 0,78 | 3,12 | 12,5 | 12,5 | 3,12 | 6,25 | 6,25 | 50 |
| 41 | 3,12 | 1,56 | 1,56 | 1,56 | 3,12 | 1,56 | 3,12 | 12,5 | 3,12 | 3,12 | 3,12 | 3,12 |
| 31 | 12,5 | 3,12 | 3,12 | 25 | 3,12 | 6,25 | 12,5 | 25 | 12,5 | 12,5 | 12,5 | 2,5 |
| A | 1,56 | 6,25 | 6,25 | 6,25 | 1,56 | 3,12 | 12,5 | 12,5 | 3,12 | 12,5 | 12,5 | 12,5 |

Beispiel F

Bestimmung der letalen Dosis von (1-(2-(2,4-Dichlorphenyl)-2-(3-(4-chlorphenylthio)-propylamid)ethyl-1-H-imidazol-dihydrochlorid (Verbindung Nr. 19) bei Mäusen und Ratten bei einmaliger Gabe.
Jeweils vier Gruppen von weiblichen und männlichen Tieren wurden oral Dosen von 0,500, 1000 und 3000 mg/kg Körpergewicht verabreicht.
(Kontrolle: zweifach destilliertes Wasser)
Folgende klinische Symptome wurden beobachtet.

Mäuse:Inaktivität, Konvulsionen
Ratten:Inaktivität, gekräuseltes Fell, Konvulsionen

| Mäuse | $LD_{100}$ | |
|---|---|---|
| weibl. | >1000 mg/kg | <3000 mg/kg |
| männl. | >500 mg/kg | <1000 mg/kg |
| Ratten | | |
| weibl. | >500 mg/kg | <1000 mg/kg |
| männl. | >1000 mg/kg | <3000 mg/kg |

Beispiel G

Bestimmung der $LD_{50}$ von (1-(2-(2,4-Dichlorphenyl)-2-(3-(4-chlorphenylthio)propylamid)ethyl-1-H-imidazol-dihydrochlorid (Verbindung Nr. 19) an Mäusen und Ratten durch i. v. Gabe jeweils 5 Gruppen von weiblichen und männlichen Tieren wurden 0 (0,9 % NACl-Lösung) 12,5, 25,0, 50,0 und 100 mg/kg Körpergewicht i.v. injiziert.
Folgende klinische Symptome wurden beobachtet
Mäuse:Inaktivität, necrotischer Schwanz
Ratten:Inaktivität, necrotischer Schwanz, Konvulsionen

| | $LD_{50}$ |
|---|---|
| Mäuse | |
| weibl. | 84,1 (20,8 - 340,0) mg/kg |
| männl. | 42,0 (13,9 - 127,5) mg/kg |
| Ratten | |
| weibl. | 56,1 (35,4 - 89,1) mg/kg |
| männl. | 70,7 mg/kg |

Beispiel H

Verbindung Nr. 19 wurde auf ihr Potential Genmutationen zu verursachen in fünf Salmonella typhimurum-Stammes TA 1535, TA 1537, TA 1538, TA 98 und TA 100 untersucht.
Folgende Konzentrationen wurden getestet, sowohl mit metabolischem Aktivator (S 9 mix) als auch ohne metabolischen Aktivator.
I: 10, 33,3, 100,0, 333,3, 1000 µg/Platte
II: 3,3, 10, 33,3, 100, 333,3 µg/Platte
Es wurde keinerlei mutagene Aktivität beobachtet, weder mit noch ohne metabolischen Aktivator.

## Ansprüche

1. Imidazolderivate der allgemeinen Formel I des Formelblattes, in welcher Ar gegebenenfalls durch Halogen, Niederalkyl oder Niederalkoxy substituiertes Phenyl, Biphenylyl, Naphthyl oder Thienyl, $R_1$ Wasserstoff oder Niederalkyl, Alk geradkettiges oder verzweigtes Alkylen mit 1 bis 10 Kohlenstoffatomen, Y Sauerstoff, Schwefel, Sulfinyl oder Sulfonyl, n eine der Zahlen 0, 1 oder 2, Z Schwefel oder Sulfinyl, m die Zahl 0 oder 1, wobei, wenn Y Schwefel, Sulfinyl oder Sulfonyl bedeutet, m die Zahl 0und wenn Y Sauerstoff bedeutet, m die Zahl 1 ist, $R_2$ Cyclohexyl, gegebenenfalls durch Hydroxy, Halogen, Trifluormethyl, Niederalkyl oder Niederalkoxy substituiertes Phenyl oder Naphthyl, Biphenylyl oder Pyridyl bedeuten und deren pharmazeutisch annehmbare Säureadditionssalze.

2. Imidazolderivate nach Anspruch 1 der allgemeinen Formel I, in der $R_1$ ein Wasserstoffatom oder den Methylrest, Ar 2,4-Dichlorphenyl oder 4-Chlorphenyl, Alk geradkettiges oder verzweigtes Alkylen mit 1 bis 6 C-Atomen, Y Schwefel, Sulfinyl oder Sulfonyl, Z Schwefel oder Sulfinyl, und $R_2$ Cyclohexyl, Phenyl, 4-Methylphenyl, 4-Fluorphenyl, 4-Chlorphenyl, 4-Bromphenyl, 2,6-Dichlorphenyl oder Naphthyl bedeuten.

3. Imidazolderivate nach Anspruch 1, ausgewählt aus
1-(2-(2,4-Dichlorphenyl)-2-(3-(4-chlorphenylthio)propylamino))ethyl-1H-imidazol
1-(2-(2,4-Dichlorphenyl)-2-(2-(2-(4-chlorphenylthio)ethoxy)ethylamino))ethyl-1H-imidazol
1-(2-(2,4-Dichlorphenyl)-2-(2-(4-bromphenylthio)ethylamino))ethyl-1H-imidazol
1-(2-(2,4-Dichlorphenyl)-2-(3-cyclohexylthiopropylamino))ethyl-1H-imidazol
1-(2-(2,4-Dichlorphenyl)-2-(3-(2-naphthylthio)propylamino))ethyl-1H-imidazol
1-(2-(2,4-Dichlorphenyl)-2-(N-methyl-N-(3-(4-chlorbenzylthio)propylamino)))ethyl-1H-imidazol

4. Verfahren zur Herstellung der Verbindungen der allgemeinen Formel I, dadurch gekennzeichnet, daß man Verbindungen der allgemeinen Formel II des Formelblattes, worin Ar obige Bedeutung hat, mit Verbindungen der allgemeinen Formel III des Formelblattes, worin $R_2$, Z, m, n, Y und Alk obige Bedeutung haben, gegebenenfalls in Gegenwart eines inerten Verdünnungsmittels umsetzt, die erhaltenen Iminoverbindungen der allgemeinen Formel IV des Formelblattes, in welcher Ar, Alk, Y, Z, $R_2$, n und m obige Bedeutung haben, gegebenenfalls in Gegenwart eines inerten Verdünnungsmittels reduziert und gewünschtenfalls die erhaltenen Verbindungen der Formel I, in denen $R_1$ Wasserstoff bedeutet, durch übliche Alkylierungsmethoden in Verbindung der Formel I, in denen $R_1$ Niederalkyl bedeutet, überführt.

5. Verfahren zur Herstellung der Verbindungen der allgemeinen Formel I, dadurch gekennzeichnet, daß man eine Verbindung der allgemeinen Formel II des Formelblattes, mit einem Aminoalkanol der allgemeinen Formel V des Formelblattes, umsetzt, die als Umsetzungsprodukt erhaltene Iminoverbindung der allgemeinen Formel VI des Formelblattes, reduziert, die erhaltene Hydroxyalkylaminoverbindung der allgemeinen Formel VII des Formelblattes, in die entsprechende Halogenalkylaminoverbindung der allgemeinen Formel VIII des Formelblattes, überführt, diese mit einer Verbindung der allgemeinen Formel IX des Formelblattes, umsetzt und gewünschtenfalls die erhaltenen Verbindungen der Formel I, in denen $R_1$ Wasserstoff bedeutet, durch übliche Alkylierungsmethoden in Verbindungen der Formel I, in denen $R_1$ Niederalkyl bedeutet, überführt, wobei in obigen Formeln V bis IX Ar, Alk, Y, Z, $R_2$, X, n und m die für Formel I angegebene Bedeutung haben und Hal für Halogen steht.

6. Antimykotische Mittel, gekennzeichnet durch den Gehalt an mindestens einer Verbindung der allgemeinen Formel I nach Anspruch 1.

7. Verfahren zur Herstellung von antimykotischen Mitteln, dadurch gekennzeichnet, daß man Verbindungen der Formel I mit pharmazeutisch annehmbaren Träger-und/oder Hilfsstoffen vermischt.

8. Verwendung von Verbindungen der Formel I zur Herstellung von antimykotischen Mitteln.

Patentansprüche für folgende Vertragsstaat : ES

1. Verfahren zur Herstellung der Verbindungen der allgemeinen Formel I, dadurch gekennzeichnet, daß man Verbindungen der allgemeinen Formel II des Formelblattes, worin Ar obige Bedeutung hat, mit Verbindungen der allgemeinen Formel III des Formelblattes, worin $R_2$, Z, m, n, Y und Alk obige Bedeutung haben, gegebenenfalls in Gegenwart eines inerten Verdünnungsmittels umsetzt, die erhaltenen Iminoverbindungen der allgemeinen Formel IV des Formelblattes, in welcher Ar, Alk, Y, Z, $R_2$, n und m obige Bedeutung haben, gegebenenfalls in Gegenwart eines inerten

Verdünnungsmittels reduziert und gewünschtenfalls die erhaltenen Verbindungen der Formel I, in denen $R_1$ Wasserstoff bedeutet, durch übliche Alkylierungsmethoden in Verbindung der Formel I, in denen $R_1$ Niederalkyl bedeutet, überführt.

2. Verfahren zur Herstellung der Verbindungen der allgemeinen Formel I, dadurch gekennzeichnet, daß man eine Verbindung der allgemeinen Formel II des Formelblattes,
mit einem Aminoalkanol der allgemeinen Formel V des Formelblattes,
umsetzt, die als Umsetzungsprodukt erhaltene Iminoverbindung der allgemeinen Formel VI des Formelblattes,
reduziert, die erhaltene Hydroxyalkylaminoverbindung der allgemeinen Formel VII des Formelblattes,
in die entsprechende Halogenalkylaminoverbindung der allgemeinen Formel VIII des Formelblattes,
überführt, diese mit einer Verbindung der allgemeinen Formel IX des Formelblattes,
umsetzt und gewünschtenfalls die erhaltenen Verbindungen der Formel I, in denen $R_1$ Wasserstoff bedeutet, durch übliche Alkylierungsmethoden in Verbindungen der Formel I, in denen $R_1$ Niederalkyl bedeutet, überführt, wobei in obigen Formeln V bis IX Ar, Alk, Y, Z, $R_2$, X, n und m die für Formel I angegebene Bedeutung haben und Hal für Halogen steht.

3. Verfahren nach Anspruch 1, dadurch gekennzeichnet, daß die Umsetzung der Verbindungen der allgemeinen Formeln II und III des Formelblattes in einem inerten Verdünnungsmittel erfolgt.

4. Verfahren nach Anspruch 1, dadurch gekennzeichnet, daß die Reduktion in Gegenwart eines komplexen Metallhydrids erfolgt.

5. Verfahren nach Anspruch 4, dadurch gekennzeichnet, daß als Reduktionsmittel Natriumborhydrid eingesetzt wird.

6. Verfahren nach Anspruch 2, dadurch gekennzeichnet, daß die Umsetzung der Verbindungen der allgemeinen Formeln II und V des Formelblattes in einem organischen Verbindungs-mittel bei Rückflußtemperaturen des verwendeten Verdünnungsmittels erfolgt.

7. Verfahren nach Anspruch 2, dadurch gekennzeichnet, daß die Reduktion unter Zugabe von Natriumborhydrid durchgeführt wird.

Patentansprüche für folgenden Vertragsstaat : GR

1. Imidazolderivate der allgemeinen Formel I des Formelblattes,
in welcher Ar gegebenenfalls durch Halogen, Niederalkyl oder Niederalkoxy substituiertes Phenyl, Biphenylyl, Naphthyl oder Thienyl, $R_1$ Wasserstoff oder Niederalkyl, Alk geradkettiges oder verzweigtes Alkylen mit 1 bis 10 Kohlenstoffatomen, Y Sauerstoff, Schwefel, Sulfinyl oder Sulfonyl, n eine der Zahlen 0, 1 oder 2, Z Schwefel oder Sulfinyl, m die Zahl 0 oder 1, wobei, wenn Y Schwefel, Sulfinyl oder Sulfonyl bedeutet, m die Zahl 0und wenn Y Sauerstoff bedeutet, m die Zahl 1 ist, $R_2$ Cyclohexyl, gegebenenfalls durch Hydroxy, Halogen, Trifluormethyl, Niederalkyl oder Niederalkoxy substituiertes Phenyl oder Naphthyl, Biphenylyl oder Pyridyl bedeuten und deren pharmazeutisch annehmbare Säureadditionssalze.

2. Imidazolderivate nach Anspruch 1 der allgemeinen Formel I, in der $R_1$ ein Wasserstoffatom oder den Methylrest, Ar 2,4-Dichlorphenyl oder 4-Chlorphenyl, Alk geradkettiges oder verzweigtes Alkylen mit 1 bis 6 C-Atomen, Y Schwefel, Sulfinyl oder Sulfonyl, Z Schwefel oder Sulfinyl, und $R_2$ Cyclohexyl, Phenyl, 4-Methylphenyl, 4-Fluorphenyl, 4-Chlorphenyl, 4-Bromphenyl, 2,6-Dichlorphenyl oder Naphthyl bedeuten.

3. Imidazolderivate nach Anspruch 1, ausgewählt aus

1-(2-(2,4-Dichlorphenyl)-2-(3-(4-chlorphenylthio)propylamino))ethyl-1H-imidazol
1-(2-(2,4-Dichlorphenyl)-2-(2-(2-(4-chlorphenylthio)ethoxy)ethylamino))ethyl-1H-imidazol
1-(2-(2,4-Dichlorphenyl)-2-(2-(4-bromphenylthio)ethylamino))ethyl-1H-imidazol
1-(2-(2,4-Dichlorphenyl)-2-(3-cyclohexylthiopropylamino))ethyl-1H-imidazol
1-(2-(2,4-Dichlorphenyl)-2-(3-(2-naphthylthio)propylamino))ethyl-1H-imidazol
1-(2-(2,4-Dichlorphenyl)-2-(N-methyl-N-(3-(4-chlorbenzylthio)propylamino)))ethyl-1H-imidazol

4. Verfahren zur Herstellung der Verbindungen der allgemeinen Formel I, dadurch gekennzeichnet, daß man Verbindungen der allgemeinen Formel II des Formelblattes,
worin Ar obige Bedeutung hat, mit Verbindungen der allgemeinen Formel III des Formelblattes,
worin $R_2$, Z, m, n, Y und Alk obige Bedeutung haben, gegebenenfalls in Gegenwart eines inerten Verdünnungsmittels umsetzt, die erhaltenen Iminoverbindungen der allgemeinen Formel IV des Formelblattes,
in welcher Ar, Alk, Y, Z, $R_2$, n und m obige Bedeutung haben, gegebenenfalls in Gegenwart eines inerten

Verdünnungsmittels reduziert und gewünschtenfalls die erhaltenen Verbindungen der Formel I, in denen $R_1$ Wasserstoff bedeutet, durch übliche Alkylierungsmethoden in Verbindung der Formel I, in denen $R_1$ Niederalkyl bedeutet, überführt.

5. Verfahren zur Herstellung der Verbindungen der allgemeinen Formel I, dadurch gekennzeichnet, daß man eine Verbindung der allgemeinen Formel II des Formelblattes,
mit einem Aminoalkanol der allgemeinen Formel V des Formelblattes,
umsetzt, die als Umsetzungsprodukt erhaltene Iminoverbindung der allgemeinen Formel VI des Formelblattes,
reduziert, die erhaltene Hydroxyalkylaminoverbindung der allgemeinen Formel VII des Formelblattes,
in die entsprechende Halogenalkylaminoverbindung der allgemeinen Formel VIII des Formelblattes,
überführt, diese mit einer Verbindung der allgemeinen Formel IX des Formelblattes,
umsetzt und gewünschtenfalls die erhaltenen Verbindungen der Formel I, in denen $R_1$ Wasserstoff bedeutet, durch übliche Alkylierungsmethoden in Verbindungen der Formel I, in denen $R_1$ Niederalkyl bedeutet, überführt, wobei in obigen Formeln V bis IX Ar, Alk, Y, Z, $R_2$, X, n und m die für Formel I angegebene Bedeutung haben und Hal für Halogen steht.

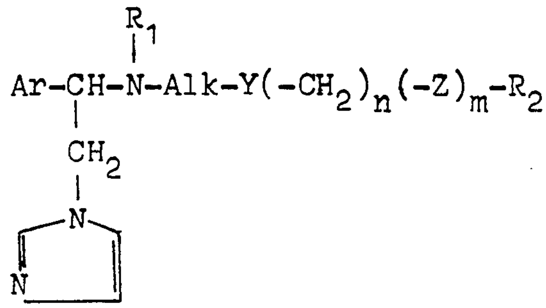

I

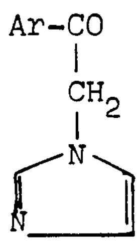

II

$R_2(-Z)_m(CH_2)_n-Y-Alk-NH_2$ III

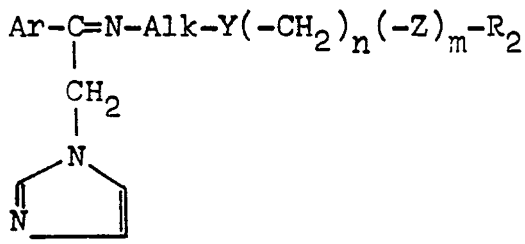

IV

$H_2N-Alk-OH$ V

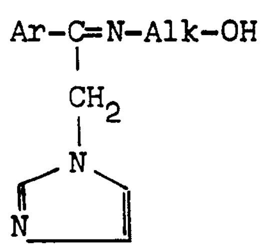

VI

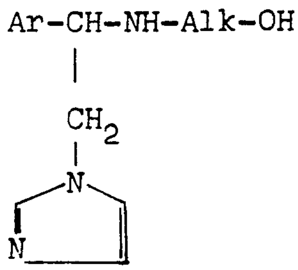

VII

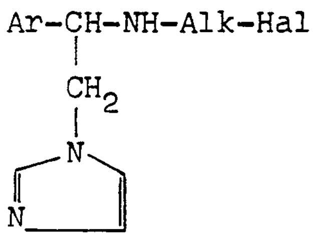

VIII

$$HY(-CH_2)_n(-Z)_m-R_2$$

IX